# EUROPÄISCHE PATENTSCHRIFT

(11) **EP 2 699 180 B1**
(45) Veröffentlichungstag und Bekanntmachung des Hinweises auf die Patenterteilung: **15.04.2015**
(21) Anmeldenummer: 13727135.9
(22) Anmeldetag: 04.06.2013
(51) Int. Cl.: A61B 17/80, A61B 17/82

(54) **MULTIDIREKTIONALE THORAXWAND-STABILISATION**
MULTIDIRECTIONAL CHEST WALL STABILIZATION
STABILISATION MULTIDIRECTIONNELLE DE LA PAROI THORACIQUE

(30) Priorität: 08.06.2012 DE 102012104978
(43) Veröffentlichungstag der Anmeldung: 26.02.2014
(73) Patentinhaber: Medxpert GmbH, 79427 Eschbach (DE)
(72) Erfinder: REISBERG, Erhard, 79427 Eschbach (DE)
(74) Vertreter: Lermer, Christoph
(86) Internationale Anmeldenummer: PCT/EP2013/061446
(87) Internationale Veröffentlichungsnummer: WO 2013/182545

(56) Entgegenhaltungen:
- DE-A1-102006 042 277
- US-A1- 2004 117 016
- US-A1- 2006 058 786
- US-A1- 2011 034 958

## Beschreibung

### TECHNISCHES GEBIET

Die vorliegende Erfindung betrifft ein erstes Implantat zur Befestigung des Implantats an einem Röhrenknochen, insbesondere an einem Rippenknochen, umfassend: eine Klammer mit einem Steg und mit sich vom Steg erstreckenden Zinken; und ein Anschlusselement zum Anschließen des Implantats an ein Verbinder-Implantat.

Es wird außerdem ein erstes Implantat zur Befestigung des Implantats an einem Röhrenknochen, insbesondere an einem Rippenknochen, beschrieben, umfassend: eine Klammer mit einem Steg, der eine Längsachse aufweist, und mit wenigstens einem ersten Zinken, einem zweiten Zinken und einem dritten Zinken, wobei sich die Zinken vom Steg quer zur Längsachse erstrecken, und der erste und der dritte Zinken relativ zur Längsachse an einer ersten Seite des Stegs angeordnet sind, und der zweite Zinken relativ zur Längsachse an einer zweiten Seite des Stegs angeordnet ist. Darüber hinaus wird ein zweites Implantat zur Verbindung zweier Verbinder-Implantate eines Implantatsystems beschrieben, umfassend: einen Steg; ein erstes Anschlusselement, und ein zweites Anschlusselement, wobei das erste Anschlusselement in einem ersten Endbereich des Stegs und das zweite Anschlusselement in einem zweiten Endbereich des Stegs angeordnet ist.

### STAND DER TECHNIK

Beim rekonstruktiven und/oder stabilisierenden Einsatz herkömmlicher Implantate am knöchernen Brustkorb sind lange Inzisionen bzw. großflächige Präparationen erforderlich. Dies führt zu einem erheblichen OP-Trauma und die Patienten müssen geraume Zeit in der Klinik verbleiben. Die Rekonvaleszenz-Phase ist relativ lange.

Stand der Technik in der Deformitäten-Chirurgie heute ist, dass nach "Ravitch" der Zugang zur knöchernen Brustwand geschaffen wird. Im überwiegenden Teil der Fälle manifestieren sich Deformitäten des Brustkorbes wie Pectus excavatum, Pectus carinatum, Pectus arcuatum, etc. im vorderen Bereich der Brustwand, da durch Fehlbildungen im Bereich der anterioren Rippenknorpel mit Auswirkungen auf die knöchernen Rippenstrukturen symmetrische oder asymmetrische zumindest kosmetisch relevante Deformitäten entstehen.

Der Zugang zur anterioren Brustwand nach Ravitch erfolgt durch eine längsaxiale Inzision mit anschließender Weichteilpräparation am Sternum oder quer dazu, submammillär. Der Eingriff ist also relativ groß und hinterlässt in der Regel eine deutlich sichtbare Narbe.

Die Druckschrift DE 10 2006 042 277 A1 offenbart ein Implantat zur Osteosynthese umfassend eine Klammer mit einem Steg und sich vom Steg erstreckenden Zinken sowie ein Anschlusselement.

### AUFGABE DER ERFINDUNG

Ausgehend davon besteht die Aufgabe der vorliegenden Erfindung darin, ein Implantat bereitzustellen, das minimalinvasive rekonstruktive und/oder stabilisierende Eingriffe am knöchernen Brustkorb ermöglicht, wobei ein flexibler Einsatz des Implantats bei unterschiedlichen anatomischen Gegebenheiten möglich sein soll.

### TECHNISCHE LÖSUNG

Diese Aufgabe wird gelöst durch ein erstes Implantat gemäß Anspruch 1. Vorteilhafte Ausgestaltungen sind Gegenstand der abhängigen Ansprüche.

In einer ersten Variante umfasst das erfindungsgemäße erste Implantat zur Befestigung des Implantats an einem Röhrenknochen, insbesondere an einem Rippenknochen: eine Klammer mit einem Steg und mit sich vom Steg erstreckenden Zinken; und ein Anschlusselement zum Anschließen des Implantats an ein Verbinder-Implantat. Die Klammer und das Anschlusselement sind erfindungsgemäß relativ zueinander um eine Achse drehbar (bzw. rotierbar bzw. um die Achse verschwenkbar) verbunden.

Auf diese Weise wird sichergestellt, dass das Implantat vielseitig, d.h. bei unterschiedlichen Gegebenheiten an unterschiedlichen Stellen des Brustkorbs, eingesetzt werden kann. Soll beispielsweise an einer bestimmten Stelle des Brustkorbs eine Befestigung bzw. Verankerung für ein Verbinder-Implantat, das insbesondere stabförmig ausgebildet ist, bzw. eine Überbrückung vorgesehen werden, so wird zunächst die Klammer des erfindungsgemäßen ersten Implantats an einer bestimmten Stelle an einem Rippenknochen befestigt. Anschließend kann das Verbinder-Implantat bzw. die Überbrückung in das Anschlusselement eingeschoben werden. Das Verbinder-Implantat wird in Längsrichtung des Anschlusselements relativ zu diesem ausgerichtet. Außerdem wird das Anschlusselement und damit das Verbinder-Implantat gegenüber der Klammer in einer bestimmten gewünschten Winkelstellung ausgerichtet bzw. relativ zur Klammer gedreht, sodass die Überbrückung einer bestimmten Strecke im Brustkorbbereich bis hin zu einem weiteren Befestigungspunkt durch das Verbinder-Implantat in beliebiger Richtung realisiert werden kann. Ob das Anschlusselement in der gewünschten Winkelstellung an der Klammer fixiert wird oder drehbar bleibt, ist, je nach den Anforderungen, unterschiedlich. Jedenfalls kann das Verbinder-Implantat relativ zur Klammer des Implantats multidirektional ausgerichtet und in beliebiger Winkelstellung (relativ zur Klammer) am Anschlusselement angebracht werden. Durch Verschieben des Verbinder-Implantats in einer Führung des Anschlusselements kann gleichzeitig die relative Ausrichtung des Verbinder-Implantats entlang der Längsachse (bzw. axialen Achse) des Anschlusselements eingestellt und in der Endstellung fixiert werden.

Im Ergebnis bleibt festzuhalten, dass die Verbindung zwischen dem Anschlusselement (=Schuh) und dem Steg um 360° rotierbar gestaltet werden kann, um Verbindungs-Implantate in beliebige Richtungen von einem Verankerungspunkt zum gegenüberliegenden Verankerungspunkt ausrichten zu können.

Vorzugsweise weist das Implantat einen Konnektor auf, der die drehbare Verbindung zwischen der Klammer und dem Anschlusselement herstellt. So kann der Konnektor drehbar mit der Klammer und/oder dem Anschlusselement verbunden sein. Der Konnektor kann durch Einpressen oder Verschrauben im Steg und/oder im Anschlusselement befestigt sein (drehfest) und die jeweils andere Komponente (Anschlusselement bzw. Steg) kann drehbar am Konnektor gelagert sein. So kann die jeweilige Komponente eine Öffnung aufweisen, durch die ein Hauptkörper des Konnektors, der zylindrisch oder hohlzylindrisch ausgebildet sein kann und/oder eine Achse bildet, hindurch geführt ist. Ein Abheben der drehbar am Konnektor angebrachten Komponente vom Konnektor bzw. von der anderen Komponente wird durch einen Rand oder Vorsprung verhindert, der sich vom Hauptkörper des Konnektors wenigstes abschnittsweise über den Rand der Öffnung der drehbar am Konnektor angebrachten Komponente erstreckt.

Vorzugsweise weist die Klammer einen Steg auf, der eine Längsachse aufweist, und wenigstens einen ersten Zinken, einen zweiten Zinken und einen dritten Zinken, wobei sich die Zinken vom Steg quer (insbesondere senkrecht) zur Längsachse erstrecken, und der erste und der dritte Zinken relativ zur Längsachse an einer ersten Seite des Stegs angeordnet sind, und der zweite Zinken relativ zur Längsachse an einer zweiten Seite des Stegs angeordnet ist. Die Zinken sind relativ zur Längsachse versetzt zueinander angeordnet. Insbesondere sind die Zinken so versetzt zueinander angeordnet, dass sie auf Lücke zueinander stehen. Auf diese Weise können die freien Enden der Zinken beim Zusammendrücken mit einem Werkzeug ineinander greifen und behindern sich nicht gegenseitig.

In einer zweiten Variante umfasst das erfindungsgemäße erste Implantat zur Befestigung des ersten Implantats an einem Röhrenknochen, insbesondere an einem Rippenknochen: eine Klammer mit einem Steg, der eine Längsachse aufweist, und wenigstens einen ersten Zinken, einen zweiten Zinken und einen dritten Zinken, wobei sich die Zinken vom Steg quer, insbesondere senkrecht, zur Längsachse erstrecken, und der erste und der dritte Zinken relativ zur Längsachse an einer ersten Seite des Stegs angeordnet sind, und der zweite Zinken relativ zur Längsachse an einer zweiten Seite des Stegs angeordnet ist. Die Zinken sind relativ zur Längsachse versetzt zueinander angeordnet. Erste und zweite Seite des Stegs liegen sich in der Regel gegenüber.

Insbesondere sind die Zinken so versetzt zueinander angeordnet, dass sie auf Lücke zueinander stehen. Der erste und der dritte Zinken können dabei so angeordnet sein, dass der zweite Zinken zwischen diese eingreift.

Insbesondere erstrecken sich die mit dem Steg verbundenen Abschnitte der Zinken vom Steg quer bzw. senkrecht relativ zur Längsachse nach außen. Die dem Steg abgewandten Endabschnitte der Zinken können darüber hinaus aus einer Ebene, die durch den Steg bestimmt wird, heraus nach unten gebogen sein, sodass das Implantat relativ einfach an einen Rippenknochen angelegt und anschließend die Zinken mittels eines Werkzeugs wenigstens teilweise um den Rippenknochen gebogen werden können. Auf diese Wiese bilden Steg und Zinken eine Klammer.

Vorzugsweise ist die Materialstärke des zweiten Zinkens (zusammen mit der Materialstärke eventueller weiterer an der zweiten Seite angeordneter und vom Werkzeug erfasster Zinken) so eingestellt, dass der Biegewiderstand der Summe der Biegewiderstände des ersten und des dritten Zinkens (zusammen mit dem Biegewiderstand eventueller weiterer an der ersten Seite angeordneter und vom Werkzeug erfasster Zinken) entspricht. Dadurch wird vermieden, dass sich die Klammer während des Befestigens am Rippenknochen im Werkzeug dreht.

Die Bandbreite an anzubietenden Klammer-Größen kann damit auf ein Minimum beschränkt werden, was durch das "Nut- und Feder-Konzept" parallel schließbarer Klammerzinken möglich ist. Die Klammer wird in der Regel dreizinkig mit versetzten Zinken gestaltet, sodass sich die Zinken bei unterschiedlich dimensionierten Rippen nicht an der Rückseite einer Rippe treffen oder überlappen, sondern parallel nebeneinander fixiert werden können. Zum Befestigen an einem Rippenknochen wird eine dreiendige Rippenklammer-Fixierzange mit einem dreiteiligen Maulteil zum Umfassen, Führen und Anbiegen der Klammer verwendet.

Mit Hilfe der Varianten des ersten Implantats ist es möglich, durch zwei kleine seitliche Inzisionen/Präparationen links und rechts des Sternums die Klammer-Implantate an den Rippen zu verankern und mittels anschließender Tunnelierung Raum für den Verbindungssteg zwischen den Klammern zu schaffen. Die Idee, über mehrere kleinere Zugänge plus Gewebetunnel Implantatbrücken einzubringen, ist auch analog für andere Rekonstruktionen / Stabilisierungen der Thoraxwand denkbar. Die Klammer-Implantate können auf kleinstem Raum an einer Rippe verankert werden.

Die im Zusammenhang mit der ersten Variante des erfindungsgemäßen ersten Implantats und der zweiten Variante des erfindungsgemäßen ersten Implantats genannten und beschriebenen Merkmale werden, ausgehend von der jeweils beanspruchten Grundform, in unterschiedlichen Kombinationen beansprucht, auch wenn diese Kombinationen nicht explizit beschrieben sind.

Ein zweites Implantat zur Verbindung zweier Verbinder-Implantate eines Implantatsystems wird beschrieben, das nicht Teil der Erfindung ist, umfassend: einen Steg; ein erstes Anschlusselement, und ein zweites Anschlusselement, wobei das erste Anschlusselement in einem ersten Endbereich des Stegs und das zweite Anschlusselement in einem zweiten Endbereich des Stegs angeordnet ist. Das erste Anschlusselement und/oder das zweite Anschlusselement ist drehbar mit dem Steg verbunden.

Die Vorteile der drehbaren Befestigung eines Anschlusselements am Steg wurden bereits im Zusammenhang mit der ersten Variante des ersten Implantats beschrieben. Diese Vorteile und Anwendungsmöglichkeiten sind auf das zweite Implantat übertragbar. Insbesondere ist die Möglichkeit einer multidirektionalen Ausrichtung zweier Verbinder-Implantate zueinander gegeben. Durch Verschieben der Verbinder-Implantate in einer Führung des ersten und/oder zweiten Anschlusselements kann gleichzeitig die relative (axiale oder Längs-)Ausrichtung des jeweiligen Verbinder-Implantats zum entsprechenden Anschlusselement eingestellt und in der Endstellung fixiert werden. Lediglich weist das zweite Implantat im Gegensatz zum ersten Implantat keine Befestigungsklammer auf.

Vorzugsweise weist das zweite Implantat einen Konnektor pro drehbarer Verbindung auf, der die drehbare Verbindung zwischen dem Steg und dem jeweiligen Anschlusselement herstellt. So kann der Konnektor drehbar mit dem Steg und/oder dem jeweiligen Anschlusselement verbunden sein. Der Konnektor kann beispielsweise durch Einpressen oder Verschrauben im Steg und/oder im Anschlusselement befestigt sein (drehfest) und die jeweils andere Komponente (Anschlusselement bzw. Steg) kann drehbar am Konnektor gelagert sein. So kann die jeweilige drehbar am Konnektor angeordnete Komponente eine Öffnung aufweisen, durch die ein Hauptkörper des Konnektors, der zylindrisch oder hohlzylindrisch ausgebildet sein kann und/oder eine Achse bildet, hindurch geführt ist. Ein Abheben der betreffenden drehbar gelagerten Komponente vom Konnektor bzw. von der anderen Komponente wird durch einen Rand oder Vorsprung verhindert, der sich vom Hauptkörper des Konnektors nach außen wenigstes abschnittsweise über den Rand der Öffnung erstreckt. Das beschriebene zweite Implantat ist ein Verbindungsteil, das bei z.B. vertikaler Ausrichtung einer Implantatbrücke sternum- oder wirbelsäulennah eine rigide winkelfixierende oder rotierbare funktionsdynamische Querverbindung zweier Verbindungsstege zueinander ermöglicht.

Insbesondere ist der Konnektor eines ersten oder zweiten Implantats, wie oben beschrieben, derart ausgebildet, dass der Steg in einer beliebigen Winkelausrichtung relativ zum Anschlusselement fixierbar ist. Diese Variante wird beispielsweise durch eine Deformierbarkeit des Konnektors, der beispielsweise als Hohlzylinder ausgebildet sein kann, realisisert. Die Deformation erfolgt beispielsweise während des Fixierens eines Verbinder-Implantats am Anschlusselement.

In einer alternativen Ausführungsform ist der Konnektor derart ausgebildet, dass das Anschlusselement beim Fixieren eines Verbinder-Implantats am Anschlusselement eine drehbare Verbindung zum Steg beibehält. Dabei ist der Konnektor beispielsweise als Vollzylinder ausgebildet, der praktisch nicht deformierbar ist bei äußeren Krafteinwirkungen, wie sie beim Fixieren des Verbinder-Implantats am Anschlusselement auftreten.

### KURZE BESCHREIBUNG DER FIGUREN

Weitere Merkmale und Vorteile der Erfindung ergeben sich aus der Beschreibung bevorzugter Ausführungsbeispiele anhand der Figuren.
- Figur 1: ist eine perspektivische Ansicht einer Ausführungsform eines ersten Implantats gemäß der vorliegenden Erfindung;
- Figur 2: ist eine perspektivische Ansicht einer zweiten Komponente des Implantats aus der Figur 1;
- Figur 3: ist eine perspektivische Ansicht einer dritten Komponente des Implantates aus der Figur 1;
- Figur 4: ist eine Schnittansicht eines Details des Implantats aus der Figur 1;
- Figur 5: ist eine perspektivische Ansicht einer Ausführungsform eines zweiten Implantats;
- Figur 6: ist eine perspektivische Ansicht einer ersten Komponente des zweiten Implantats aus der Figur 5;
- Figur 7: ist eine perspektivische Ansicht der Ausführungsform des ersten erfindungsgemäßen Implantats aus der Figur 1 in Verbindung mit einem stabförmigen Verbinder-Implantat; und
- Figur 8: ist eine perspektivische Ansicht eines Beispiels einer Thoraxstabilisierung mit Hilfe der erfindungsgemäßen Implantate.

### BESCHREIBUNG BEVORZUGTER AUSFÜHRUNGSBEISPIELE

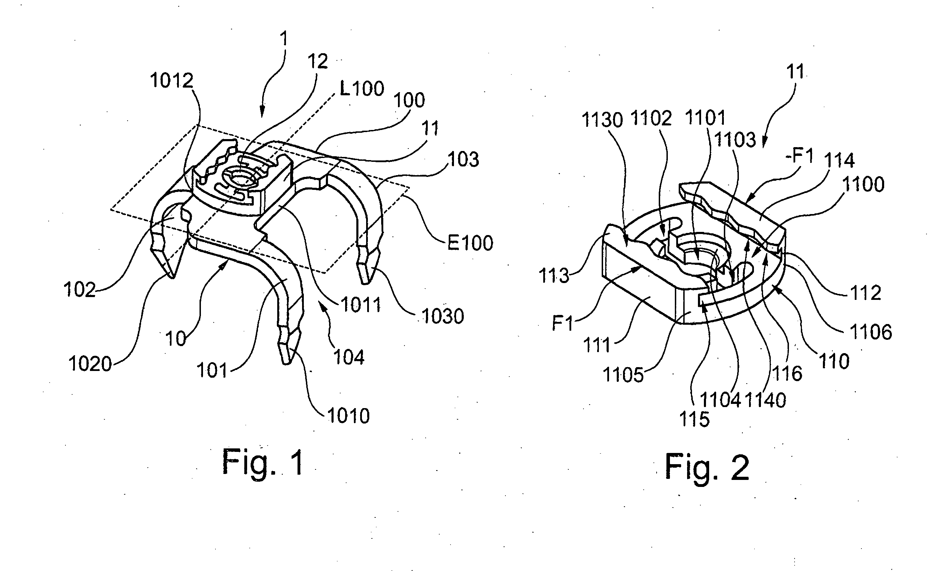

In der Figur 1 ist eine perspektivische Ansicht einer Ausführungsform eines ersten Implantats 1 gemäß der vorliegenden Erfindung dargestellt.

Das erste Implantat 1 weist eine Klammer 10 auf. Diese umfasst einen Steg 100 sowie sich vom Steg 100 beidseitig erstreckende Zinken 101, 102, 103. Der erste Zinken 101 und der dritte Zinken 103 sind (in Längsrichtung L100 des Stegs) beabstandet zueinander an einer ersten Seite 1011 des Stegs 100 angeordnet, der zweite Zinken 102 ist an einer der ersten Seite 1001 gegenüber liegenden zweiten Seite 1012 angeordnet. Die Zinken 101, 102, 103 erstrecken sich vom Steg 100 in etwa senkrecht zur Längsachse L100 des Stegs 100 nach außen weg und sind, aus der Ebene E100, die der Steg 100 aufspannt, heraus nach unten hin gebogen. Die Zinken 101, 102 und 103 sind, bezogen auf die Längsachse L100 des Stegs 100, versetzt zueinander am Steg 100 angeordnet. Der zweite Zinken 102 ist dabei auf Höhe der Lücke 104 zwischen dem ersten Zinken 101 und dem dritten Zinken 103 angeordnet. Der zweite Zinken 102 ist so angeordnet, dass er, wenn die freien Enden 1010, 1020, 1030 der Zinken 101, 102 bzw. 103 zueinander gebogen werden, auf Höhe der Lücke 104 zwischen dem ersten Zinken 101 und dem dritten Zinken 103 liegt bzw. in die Lücke 104 eingreift.

Die Materialstärke des zweiten Zinkens 102 (im vorliegenden Ausführungsbeispiel wird sie bestimmt durch die Breite des zweiten Zinkens; Einzinker) ist größer als die Materialstärke jeweils des ersten 102 und des dritte Zinkens 103 (im vorliegenden Ausführungsbeispiel bestimmt durch die Breite jeweils des ersten Zinkens 102 bzw. des dritten Zinkens 103; erster Zinken 101 und dritter Zinken 103 bilden einen Doppelzinker). Die Materialstärken bzw. Breiten sind so gewählt, dass der Widerstand des ersten und dritten Zinkens 101 und 103 in Summe so groß ist wie der Widerstand des zweiten Zinkens 102, wenn der erste und dritte Zinken 101 und 103 mit einem Werkzeug zum zweiten Zinken 102 hin gebogen werden, um die Klammer 10 an einem Rippenknochen festzuklammern. Dadurch wird ein Verkippen der Klammer 10 im Werkzeug während des Befestigens der Klammer 10 am Rippenknochen verhindert. Als Werkzeug kann insbesondere eine Biegezange verwendet werden, deren Anzahl von Maulteilen der der Anzahl der Zinken entspricht (hier also Biegezange mit drei Maulteilen). Im Allgemeinen ist die Summe der Materialwiderstand der an der ersten Seite angeordneten Zinken so groß wie die Summe der Materialstärken/-widerstände der an der zweiten Seite angeordneten Zinken (soweit die jeweiligen Zinken vom Werkzeug erfasst werden). Bei Verwendung einer dreiendigen Rippenklammer-Fixierzange ist damit der Materialwiderstand beider Seiten (Einzinker und Doppelzinker) beim Biegen identisch, ein unkontrolliertes Kippen der Klammer im Zangenmaul wird vermieden. Ferner ist ein breiterer Einzinker auch dadurch vorteilhaft, da sich die Auflagefläche an der Rippe vergrößert und so eine bessere Rotationsstabilität erreicht wird.

Als zweite Komponente weist das erste Implantat 1 ein Anschlusselement 11 auf. Dieses ist zur Aufnahme und Befestigung eines stabförmigen Verbinder-Implantats (nicht dargestellt) ausgebildet. Erfindungsgemäß ist das Anschlusselement 11 drehbar um eine Achse, die in etwa senkrecht zur vom Steg 100 aufgespannten Ebene E100 steht, am Steg 100 befestigt.

Die drehbare Verbindung zwischen dem Steg 100 der Klammer 10 und dem Anschlusselement 11 wird mit Hilfe einer dritten Komponente des Implantats 1 hergestellt. Die dritte Komponente ist im vorliegenden Ausführungsbeispiel als Zylinder-Konnektor 12 ausgebildet. Dieser ist einerseits in einer Öffnung des Stegs 100 fixiert, andererseits bildet er eine Achse, an der das Anschlusselement 11 drehbar gelagert ist.

In der Figur 2 ist das Anschlusselement 11 genauer dargestellt. Das Anschlusselement 11 weist einen Grundkörper 110 mit einer Führungsoberfläche 1100 auf, die von einer Öffnung 1101, einem ersten Durchbruch 1102 und einem zweiten Durchbruch 1103 durchbrochen ist. Am der Führungsfläche 1100 zugewandten Rand der in etwa zylindrischen Öffnung 1101 ist wenigstens abschnittsweise eine ringförmige Nut 1104 ausgebildet.

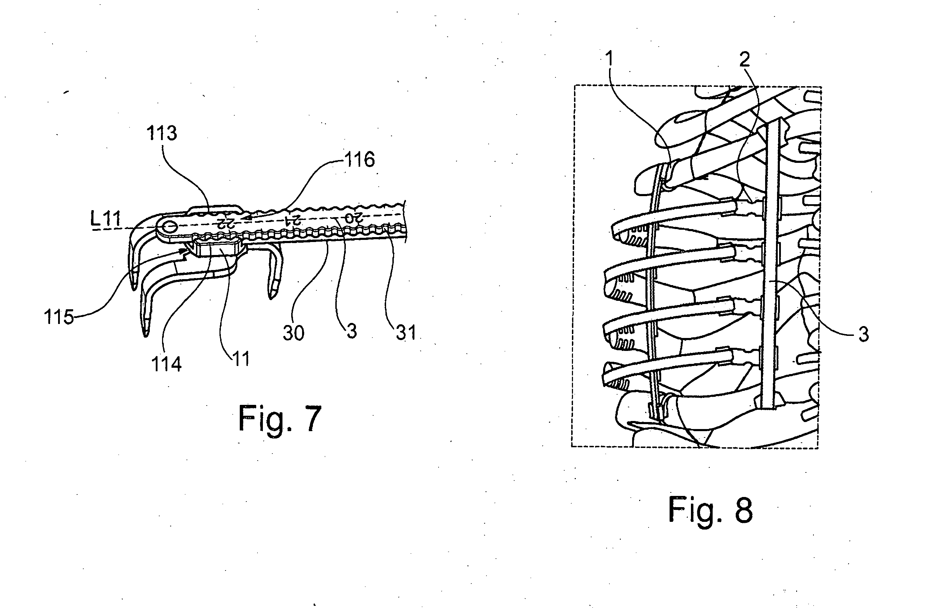

Darüber hinaus weist das Anschlusselement 11 einen sich wenigstens abschnittsweise entlang einer ersten Seite 1105 des Anschlusselements 11 aus der Führungsoberfläche 1100 hinaus nach oben erstreckenden ersten Führungssteg 111 und einen sich wenigstens abschnittsweise entlang einer zweiten Seite 1106 des Anschlusselements 11 aus der Führungsoberfläche 1100 hinaus nach oben erstreckenden zweiten Führungssteg 112 auf. Die Führungsstege 111 und 112 bilden seitliche Begrenzungen bzw. Führungen für ein auf der Führungsoberfläche 1100 des Grundkörpers 110 geführtes bzw. gleitendes Verbinder-Implantat 3 (vgl. Figur 7).

An der der Führungsoberfläche 1100 abgewandten Seite des ersten Führungsstegs 111 ist ein sich in etwa parallel zur Führungsoberfläche 1100 in Richtung des zweiten Führungsstegs 112 erstreckender erster Vorsprung 113 ausgebildet. Das freie Ende 1130 des ersten Vorsprungs 113, das dem zweiten Führungssteg 112 zugewandt ist, ist mit einer zahnartigen Kontur versehen.

Entsprechend ist an der der Führungsoberfläche 1100 abgewandten Seite des zweiten Führungsstegs 112 ein sich in etwa parallel zur Führungsoberfläche 1100 in Richtung des ersten Führungsstegs 111 erstreckender zweiter Vorsprung 114 ausgebildet. Das freie Ende 1140 des zweiten Vorsprungs 114, das dem ersten Führungssteg 111 zugewandt ist, ist ebenfalls mit einer zahnartigen Kontur versehen. Dies bedeutet, dass sich die freien Enden des ersten Vorsprungs 113 und des zweiten Vorsprungs 114 gegenüber liegen und zueinander ausgerichtet sind. Auch die zahnartigen Konturen des ersten Vorsprungs 113 und des zweiten Vorsprungs 114 liegen sich damit gegenüber und sind einander zugewandt.

Der erste Führungssteg 111 bildet zusammen mit dem ersten Vorsprung 113 eine erste Führungsnut 115 aus, der zweite Führungssteg 112 bildet zusammen mit dem zweiten Vorsprung 114 eine zweite Führungsnut 116 aus. Insgesamt bilden die Führungsoberfläche 1100, die Stege 111, 112 und die Vorsprünge 113, 114 eine Führung aus, in der ein stabförmigen Verbinder-Implantat (nicht dargestellt) mit schwalbenschwanzartige Struktur parallel zur Längsachse L110 des Grundkörpers 110 geführt werden, jedoch keine anderen translatorischen bzw. linearen Bewegungen in weiteren Dimensionen relativ zum Anschlusselement 11 durchführen kann.

Bei einem Zusammendrücken der Vorsprünge 113 und 114 und damit der sich gegenüber liegenden zahnartigen Konturen 115 und 116 deformiert sich der Grundkörper 110. Dies wird u.a. ermöglicht durch die im Querschnitt T-förmigen Durchbrüche 1102 und 1103, die jeweils mit der Öffnung 1101 in Verbindung stehen.

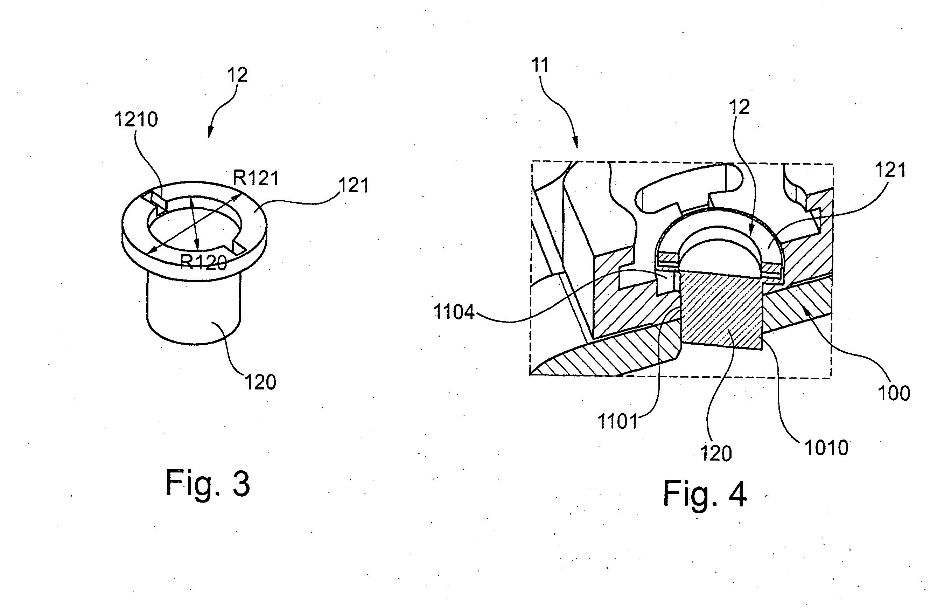

Die Figur 3 zeigt als dritte Komponente des Implantats 1 den Zylinder-Konnektor 12, der die Klammer 10 mit dem Anschlusselement 11 verbindet.

Der Zylinder-Konnektor 12 weist einen Grundkörper 120 mit zylindrischer Außenfläche auf. Im vorliegenden Ausführungsbeispiel ist der Grundkörper 120 zylindrisch. Er kann jedoch alternativ dazu auch hohlzylindrisch bzw. ringförmig ausgebildet sein (nicht dargestellt).

An einer ersten Stirnseite ist ein sich über den Radius R120 des Grundkörpers 120 hinaus nach außen erstreckender kreisförmiger Vorsprung bzw. Rand 121ausgebildet (R120 < R121). An der dem Grundkörper 120 abgewandten Seite ist ein Schraubenschlitz 1210 zum Festschrauben des Zylinder-Konnektors 12 in der Öffnung 1101 des Anschlusselements 11 ausgebildet.

In der dargestellten Ausführungsform bleibt, wenn ein Verbindungs-Implantat im Anschlusselement durch Zusammendrücken der Führungsstege axial fixiert wird, die Rotierbarkeit des Anschlusselements weiterhin gegeben. Dies kann von Vorteil sein, wenn eine gewisse Flexibilität der Stützkonstruktion erwünscht ist, um funktionalen anatomischen Anforderungen Rechnung zu tragen (rotierbare, funktionsdynamische Verbindung), beispielsweise um das Atmen des Patienten zu erleichtern.

Das Anschlusselement und damit die Stegverbindung kann in einer nicht dargestellten anderen Ausführungsform in definierter Ausrichtung des Anschlusselements relativ zur Klammer fixiert werden (rigide, winkelfixierende Verbindung). Dazu könnte der Konnektor beispielsweise einen hohlzylindrischen Grundkörper aufweisen, der, wenn ein Verbindungs-Implantat im Anschlusselement durch Zusammendrücken der Führungsstege axial fixiert wird, gleichzeitig deformiert, d.h. in der Öffnung verpresst wird, und so seine Rotierbarkeit aufgrund der Deformation des zylindrischen Grundkörpers verliert.

Die Figur 4 zeigt eine Schnittansicht eines Details des ersten Implantats 1. Der Zylinder-Konnektor 12 ist in die Öffnung 1101 des Anschlusselements 11 eingeführt, wobei der Rand 121 in die Nut 1104 eingreift. Da die Außenkontur des Grundkörpers 120 und des Rands 121 im Querschnitt kreisförmig sind, ist das Anschlusselement 11 drehbar um die Achse relativ zum bzw. am Zylinder-Konnektor 12 gelagert. Die Nut 1104 und die Öffnung 1101 sind wenigstens abschnittsweise komplementär zur Außenkontur des Grundkörpers 120 bzw. des Rands 121 ausgebildet und bilden somit eine Art Lager für den Zylinder-Konnektor 12. (bzw. der Zylinder-Konnektor bildet ein Lager für das Anschlusselement).

Andererseits greift die dem Rand 121 gegenüber liegende Seite des Grundkörpers 120 des Zylinder-Konnektors 12 in eine im Steg 100 der Klammer 10 ausgebildete Befestigungsöffnung ein und ist darin drehfest befestigt, beispielsweise durch Einpressen oder Verschrauben (in letzterem Fall weist der Grundkörper 120 des Zylinder-Konnektors 12 an seiner Außenseite ein Außengewinde auf, das in ein passendes Innengewinde der Befestigungsöffnung eingreift). Der Zylinder-Konnektor 12 kann jedoch auch drehbar mit der Klammer 10 verbunden sein. Sind die Komponenten 10, 11 und 12 des Implantats 1 zusammengefügt, sind jedenfalls das Anschlusselement 11 und die Klammer 10 drehbar relativ aneinander befestigt.

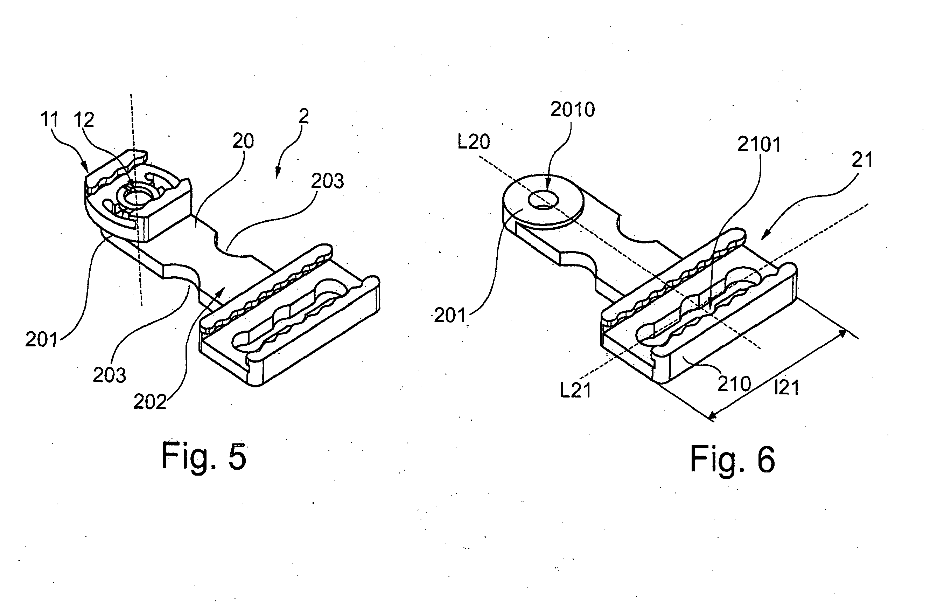

In der Figur 5 ist eine perspektivische Ansicht eines zweiten Implantats 2 dargestellt.

Das zweite Implantat 2 weist einen Hauptkörper/Steg 20 auf, in dessen erstem Endbereich 201 ein erstes Anschlusselement 11, wie in der Figur 2 dargestellt und in dem Zusammenhang beschrieben, befestigt ist. Das Anschlusselement 11 ist am Hauptkörper 20 mittels eines Zylinder-Konnektors 12, wie in der Figur 4 dargestellt und in dem Zusammenhang beschrieben, drehbar um eine Achse, die (im Wesentlichen) senkrecht zur Oberfläche des Hauptkörpers 20 ausgerichtet ist, befestigt.

Der Hauptkörper/Steg 20 erstreckt sich entlang einer Längsachse L20 (vgl. Figur 6) und weist einen ersten Endbereich 201 und einen dem gegenüber liegenden zweiten Endbereich 202 auf. Im ersten Endbereich 201 ist eine Öffnung 2010 (entsprechend der Öffnung im ersten Implantat, 1) ausgebildet, in der der Zylinder-Konnektor 12 befestigt ist, wie im Zusammenhang mit dem ersten Implantat 1 beschrieben.

Am zweiten Endbereich 202 des zweiten Implantats 2 ist ein zweites Anschlusselement 21, ähnlich dem im Zusammenhang mit dem ersten Implantat 1 beschriebenen Anschlusselement 11, ausgebildet. Um unnötige Wiederholungen zu vermeiden, werden lediglich die Unterschiede zur im Zusammenhang mit der Figur 2 beschriebenen Ausführungsform dargelegt. Das zweite Anschlusselement 21 ist starr mit dem Hauptkörper 20 verbunden. Demnach weist es nicht, wie das erste Anschlusselement 11 aus der Figur 2, eine zentrale Öffnung 1101 für den Eingriff eines Zylinder-Konnektors 12 auf. Der Grundkörper 210 weist dagegen einen Durchbruch 2101 auf, dessen Breite entlang der Längsachse L21 variiert. Dieser hat dieselbe Funktion wie die Durchbrüche 1102 und 1103 des im Zusammenhang mit dem ersten Implantat 1 beschriebenen Anschlusselements 11.

Etwa mittig ist beidseitig der Längsachse L20 jeweils eine Aussparung 203 am Hauptkörper 20 ausgebildet. Die Aussparungen (Taillierungen) 203 liegen im Stegbereich in etwa mittig und sollen ein Biegen während einer Operation ohne den Einsatz von Werkzeugen ermöglichen.

Ferner weist das quer zum Hauptkörper/Steg 20 verlaufende zweite Anschlusselement 21 eine Länge 121 auf derart, dass beim Überlappen mit einem etwa senkrecht dazu ausgerichteten Verbinder-Implantat 3 (vgl. Figur 8) stets ein Abschnitt zugänglich bleibt, der es erlaubt, ein weiteres Verbinder-Implantat 3 mit einem Werkzeug (z.B. Presszange) in Längsrichtung L21 des Anschlusselements 21 zu fixieren.

Die Figur 7 zeigt ein erstes Implantat 1, in dessen Führungsnuten 115, 116 ein stabförmig ausgebildetes Verbinder-Implantat 3 eingeführt und entlang der Längsachse L11 des Anschlusselements 11 verschiebbar ist.

Das Verbinder-Implantat 3 hat im Querschnitt eine schwalbenschwanzartige Struktur mit einer senkrecht zur Längsachse L11 des Anschlusselements 11 in die Nuten 115 und 116 eingreifende Grundplatte 30 und einer im Querschnitt senkrecht zur Längsachse L11 des Anschlusselements 11 schmaleren Oberplatte 31. Die Breite der Oberplatte ist etwas kleiner als der Abstand zwischen den freien Enden 1130 und 1140 des ersten Vorsprungs 113 bzw. des zweiten Vorsprungs 114. Soll allerdings das Verbinder-Implantat 3 mittels eines Werkzeugs in einer bestimmten Position in Längsrichtung L11 fixiert werden, so wird der Abstand zwischen dem ersten Vorsprung 113 und dem zweiten Vorsprung 114 verringert, sodass die Zahnstrukturen, die an den freien Enden 1130 und 1140 des ersten Vorsprungs 113 bzw. des zweiten Vorsprungs 114 ausgebildet sind, in entsprechende komplementäre Zahnstrukturen 31, die an den Außenseiten der Oberplatte ausgebildet sind, eingreifen. Die komplementären Zahnstrukturen 31 sind nach außen gerichtet und jeweils eine der Zahnstrukturen liegt dem ersten Vorsprung 113 bzw. dem zweiten Vorsprung 114 gegenüber. Auf diese Weise ist eine exakte relative Positionierung und Fixierung des Verbinder-Implantats 3 relativ zum ersten Implantat 1 möglich.

Die Krafteinwirkung und -richtung, die beim Fixieren durch das Werkzeug ausgeübt wird, ist in der Figur 2 durch die Pfeile F1 bzw. -F1 gekennzeichnet. In gleicher Weise kann ein Verbinder-Implantat 3 mit einem zweiten Anschlusselement 21 des zweiten Implantats 2 verbunden werden.

Die Figur 8 zeigt eine Anwendung, bei der erste Implantate 1 gemäß der Erfindung, zweite Implantate 2 und Verbinder-Implantate 3 zum Einsatz kommen.

Die Verbinder-Implantate 3 sind an Anschlusselementen der Implantate 1 und 2 fixiert. Die Darstellung zeigt eine weitere Möglichkeit, die die Erfindung bietet, nämlich eine "verlikale Stabilisierung" parallel zum Sternum oder zur Wirbelsäule bei Indikationen, bei welchen kein Rippenansatz existiert (z. B. Poland-Syndrom) oder infolge einer Tumorentfernung (extrem sternum- oder wirbelsäulennah) ein Rippenendstück entfernt werden musste. In dem dargestellten Thorax-Modell ist eine (doppelte) vertikale Stabilisierung wirbelsäulennah sowie defektmittig bei Verwendung von vier horizontalen Implantatbrücken dargestellt. In den Endbereichen ist die Überbrückung mittels erster Implantate 1 gemäß der Erfindung an den Abschlüssen der Rippen befestigt bzw. festgeklammert. Das andere Ende der horizontalen Überbruckung ist mittels zweiter Implantate 2 an einer vertikalen Überbrückung befestigt. Dadurch entsteht eine solide Gitterkonstruktion, die jedoch durch die rotierbaren Anschlüsselemente der Implantate 1 und 2 funktionelle Bewegungen des Brustkorbes zulässt.

## Patentansprüche

1. Erstes Implantat (1) zur Befestigung an einem Röhrenknochen, insbesondere an einem Rippenknochen, umfassend: eine Klammer (10) mit einem Steg (100) und mit sich vom Steg (100) erstreckenden Zinken (101, 102, 103); und ein erstes Anschlusselement (11) zum Anschließen des ersten Implantats (1) an ein Verbinder-Implantat (3), **dadurch gekennzeichnet, dass**
die Klammer (10) und das erste Anschlusselement (11) um eine Achse relativ zueinander drehbar miteinander verbunden sind.

2. Erstes Implantat (1) nach Anspruch 1,
**dadurch gekennzeichnet, dass**
das erste Implantat (1) einen Konnektor (12) aufweist, der die drehbare Verbindung zwischen der Klammer (10) und dem ersten Anschlusselement (11) herstellt.

3. Erstes Implantat (1) nach Anspruch 2,
**dadurch gekennzeichnet, dass**
die Klammer (10) und/oder das erste Anschlusselement (11) eine Öffnung aufweisen, durch die wenigstens ein Abschnitt des Konnektors (12) zur Ausbildung einer Drehachse hindurch geführt ist.

4. Erstes Implantate (1) nach einem der vorhergehenden Ansprüche,
**dadurch gekennzeichnet, dass**
die Klammer (10) einen Steg (100) aufweist, der eine Längsachse (L100) aufweist, und wenigstens einen ersten Zinken (101), einen zweiten Zinken (102) und einen dritten Zinken (103), wobei sich die Zinken vom Steg (100) quer zur Längsachse (L100) erstrecken, und der erste Zinken (101) und der dritte Zinken (103) relativ zur Längsachse (L100) an einer ersten Seite (1011) des Stegs (100) angeordnet sind, und der zweite Zinken (102) relativ zur Längsachse (L100) an einer zweiten Seite (1012) des Stegs (100) angeordnet ist, wobei die Zinken relativ zur Längsachse versetzt zueinander angeordnet sind.

5. Erstes Implantat (1) nach Anspruch 4,
**dadurch gekennzeichnet, dass**
die Zinken so versetzt zueinander angeordnet sind, dass sie auf Lücke zueinander stehen.

6. Erstes Implantat (1) nach einem der vorhergehenden Ansprüche 4 bis 5,
**dadurch gekennzeichnet, dass** sich die mit dem Steg verbundenen Abschnitte der Zinken (101, 102, 103) vom Steg quer bzw. senkrecht relativ zur Längsachse (L100) nach außen erstrecken.

7. Erstes Implantat (1) nach einem der vorhergehenden Ansprüche 4 bis 6,
**dadurch gekennzeichnet, dass**
der Biegewiderstand des oder der an der ersten Seite (1011) des Stegs (100) angeordneten und während eines Befestigungsvorgangs gemeinsam von einem Werkzeug umgriffenen Zinken dem Biegewiderstand des oder der an der zweiten Seite (1012) des Stegs (100) angeordneten und während eines Befestigungsvorgangs gemeinsam von einem Werkzeug umgriffenen Zinken entspricht.

8. Erstes Implantat (1) nach einem der vorhergehenden Ansprüche 2 bis 7,
**dadurch gekennzeichnet, dass**
der Konnektor (12) derart ausgebildet ist, dass der Steg (100, 20) in einer beliebigen Winkelausrichtung relativ zum ersten Anschlusselement (11) fixierbar ist.

9. Erstes Implantat (1) nach einem der vorhergehenden Ansprüche 2 bis 8,
**dadurch gekennzeichnet, dass**
der Konnektor (12) derart ausgebildet ist, dass das erste Anschlusselement (11) beim Fixieren eines Verbinder-Implantats (3) am ersten Anschlusselement (11) eine drehbare Verbindung zum Steg (100, 20) beibehält.

10. Erstes Implantat (1) nach einem der vorhergehenden Ansprüche,
**dadurch gekennzeichnet, dass**
das erste Anschlusselement (11) einen Grundkörper (110) mit einer Führungsoberfläche (1100), einen sich wenigstens abschnittsweise entlang einer ersten Seite (1105) des ersten Anschlusselements (11) von der Führungsoberfläche (1100) aus nach oben erstreckenden ersten Führungssteg (111), und einen sich wenigstens abschnittsweise entlang einer zweiten Seite (1106) des ersten Achlusselements (11) von der Führungsoberfläche (1100) aus nach oben erstreckenden zweiten Führungssteg (112) aufweist.

11. Erstes Implantat (1) nach Anspruch 10,
**dadurch gekennzeichnet, dass**
das erste Anschlusselement (11) einen ersten Vorsprung (113) aufweist, der sich vom ersten Führungssteg (111) weg in Richtung des zweiten Führungsstegs (112) erstreckt, und einen zweiten Vorsprung (114), der sich vom zweiten Führungssteg (112) weg in Richtung des ersten Führungsstegs (111) erstreckt.

12. Erstes Implantat (1) nach Anspruch 11,
**dadurch gekennzeichnet, dass**
das dem zweiten Führungssteg (112) zugewandte freie Ende (1130) des ersten Vorsprungs (113) eine erste zahnartige Kontur (1131) aufweist, und/oder das dem ersten Führungssteg (111) zugewandte freie Ende (1140) des zweiten Vorsprungs (114) eine zweite zahnartige Kontur (1141) aufweist.

## Claims

1. First implant (1) for attaching to a tubular bone, in particular a rib bone, comprising: a clamp (10) having a fillet (100) and prongs (101, 102, 103) extending from the fillet (100); and a first connecting element (11) for connecting the first implant (1) to a connector implant (3),
**characterised by** the fact that
the clamp (10) and the first connecting element (11) are connected so as to be rotatable relative to each other about an axis.

2. First implant (1) in accordance with claim 1,
**characterised by** the fact that
the implant (1) has a connector (12) which creates the rotatable connection between the clamp (10) and the first connecting element (11).

3. First implant (1) in accordance with claim 2,
**characterised by** the fact that
the clamp (10) and/or the first connecting element (11) have an opening, though which is guided at least a section of the connector (12) for the purpose of forming an axis of rotation.

4. First implant (1) in accordance with any of the previous claims,
**characterised by** the fact that
the clamp (10) has a fillet (100) which has a longitudinal axis (L100) and at least a first prong (101), a second prong (102) and a third prong (103), wherein the prongs extend from the fillet (100) transversely to the longitudinal axis (L100), and the first prong (101) and the third prong (103) are disposed relative to the longitudinal axis (L100) on a first side (1011) of the fillet (100), and the second prong (102) is disposed relative to the longitudinal axis (L100) on a second side (1012) of the fillet (100), wherein the prongs are disposed in a staggered arrangement opposite each other relative to the longitudinal axis.

5. First implant (1) in accordance with claim 4,
**characterised by** the fact that
the prongs are disposed in a staggered arrangement such that they are laterally offset from each other.

6. First implant (1) in accordance with any of previous claims 4 to 5,
**characterised by** the fact that
the sections of the prongs (101, 102, 103) connected to the fillet extend transversely and/or perpendicularly outwards, relative to the longitudinal axis (L100).

7. First implant (1) in accordance with any of previous claims 4 to 6,
**characterised by** the fact that
the resistance to bending of the prong(s) disposed at the first side (1011) of the fillet (100) and gripped jointly by a tool during the attachment process corresponds to the resistance to bending of the prong(s) disposed at the second side (1012) of the fillet (100) and gripped jointly by a tool during the attachment process.

8. First implant (1) in accordance with any of previous claims 2 to 7,
**characterised by** the fact that
the connector (12) is configured such that the fillet (100, 20) can be immobilized in any desired angular alignment relative to the first connecting element (11).

9. First implant (1) in accordance with any of previous claims 2 to 8,
**characterised by** the fact that
the connector (12) is configured such that the first connecting element (11) retains a rotatable connection to the fillet (100, 20) while a connector implant (3) is being immobilized on the first connecting element (11).

10. First implant (1) in accordance with any of the previous claims,
**characterised by** the fact that
the first element (11) has a base body (110), with a guide surface (1100), a guide bar (111) which extends upward from the guide surface (1100) along at least section of a first side (1105) of the first connecting element (11), and a second guide bar (112) which extends upward from the guide surface (1100) along at least a section of a second side (1106) of the first connecting element (11).

11. First implant (1) in accordance with claim 10,
**characterised by** the fact that
the first connecting element (11) has a first projection (113), which extends away from the first guide bar (111) in the direction of the second guide bar (112), and a second projection (114), which extends away from the second guide bar (112) in the direction of the first guide bar (111).

12. First implant (1) in accordance with claim 11,
**characterised by** the fact that
that free end (1130) of the first projection (113) which faces the second guide bar (112) has a first tooth-like contour (1131), and/or that free end (1140) of the second projection (114) which faces the first guide bar (111) has a second tooth-like contour (1141).

## Revendications

1. Premier implant (1) pour la fixation à un os long, en particulier à un os de côte, comprenant : une agrafe (10) avec un dos (100) et avec des dents (101, 102, 103) qui s'étendent à partir du dos (100) et un premier élément de raccord (11) pour le raccord du premier implant (1) à un implant de raccord (3), **caractérisé en ce que** l'agrafe (10) et le premier élément de raccord (11) sont reliés l'un à l'autre en étant rotatifs l'un par rapport à l'autre autour d'un axe.

2. Premier implant (1) selon la revendication 1, **caractérisé en ce que** le premier implant (1) présente un connecteur (12) qui réalise la connexion rotative entre l'agrafe (10) et le premier élément de raccord (11).

3. Premier implant (1) selon la revendication 2, **caractérisé en ce que** l'agrafe (10) et/ou le premier élément de raccord (1) présente une ouverture qui est traversée par au moins une section du connecteur (12) pour former un axe de rotation.

4. Premier implant (1) selon l'une des revendications précédentes, **caractérisé en ce que** l'agrafe (10) présente un dos (100) qui présente un axe longitudinal (L100) et au moins une première dent (101), une seconde dent (102) et une troisième dent (103), cependant que les dents s'étendent à partir du dos (100) transversalement par rapport à l'axe longitudinal (L100) et que la première dent (101) et la troisième dent (103) sont placées par rapport à l'axe longitudinal (L100) sur un premier côté (1011) du dos (100) et que la seconde dent (102) est placée par rapport à l'axe longitudinal (L100) sur un second côté (1012) du dos (100), les dents étant placées en étant décalées l'une par rapport à l'autre par rapport à l'axe longitudinal.

5. Premier implant (1) selon la revendication 4, **caractérisé en ce que** les dents sont placées en étant décalées l'une par rapport à l'autre de manière à être en chicane l'une par rapport à l'autre.

6. Premier implant (1) selon l'une des revendications précédentes 4 à 5, **caractérisé en ce que** les sections des dents (101, 102, 103) qui sont reliées au dos s'étendent vers l'extérieur à partir du dos transversalement ou perpendiculairement par rapport à l'axe longitudinal (L100).

7. Premier implant (1) selon l'une des revendications précédentes 4 à 6, **caractérisé en ce que** la résistance à la flexion de la dent ou des dents placées sur le premier côté (1011) du dos (100) et entourées ensemble par un outil pendant le processus de fixation correspond à la résistance à la flexion de la dent ou des dents placées sur le second côté (1012) du dos (100) et entourées ensemble par un outil pendant le processus de fixation.

8. Premier implant (1) selon l'une des revendications précédentes 2 à 7, **caractérisé en ce que** le connecteur (12) est configuré tel que le dos (100, 20) peut être fixé dans une orientation angulaire quelconque par rapport au premier élément de raccord (11).

9. Premier implant (1) selon l'une des revendications précédentes 2 à 8, **caractérisé en ce que** le connecteur (12) est configuré tel que l'élément de raccord (11) conserve une connexion rotative avec le dos (100, 20) lors de la fixation d'un implant de raccord (3) sur le premier élément de raccord (11).

10. Premier implant (1) selon l'une des revendications précédentes, **caractérisé en ce que** le premier élément de raccord (11) présente un corps de base (110) avec une surface de guidage (1100), une première barrette de guidage (111) qui s'étend vers le haut à partir de la surface de guidage (1100) au moins par section le long d'un premier côté (1105) du premier élément de raccord (11) et une seconde barrette de guidage (112) qui s'étend vers le haut à partir de la surface de guidage (1100) au moins par section le long d'un second côté (1106) du premier élément de raccord (11).

11. Premier implant (1) selon la revendication 10, **caractérisé en ce que** le premier élément de raccord (11) présente une première saillie (113) qui s'étend en s'éloignant de la première barrette de guidage (111) en direction de la seconde barrette de guidage (112) et une seconde saillie (114) qui s'étend en s'éloignant de la seconde barrette de guidage (142) en direction de la première barrette de guidage (111).

12. Premier implant (1) selon la revendication 11, **caractérisé en ce que** l'extrémité libre (1130) de la première saillie (113) tournée vers la seconde barrette de guidage (112) présente un premier contour de type dent (1131) et/ou l'extrémité libre (1140) de la seconde saillie (114) tournée vers la première barrette de guidage (111) présente un second contour de type dent (1141).
